# EUROPEAN PATENT SPECIFICATION

(11) **EP 1 610 769 B1**
(45) Date of publication and mention of the grant of the patent: **03.12.2008**
(21) Application number: 04723638.5
(22) Date of filing: 26.03.2004
(51) Int. Cl.: A61K 9/70

(54) **DRUG DELIVERY DEVICE COMPRISING A MESH SLEEVE**
VORRICHTUNG ZUR WIRKSTOFFFREISETZUNG MIT EINER NETZHÜLSE
DISPOSITIF D'ADMINISTRATION DE MEDICAMENTS COMPRENANT UN MANCHON EN MAILLE

(30) Priority: 26.03.2003 GB 0306977
(43) Date of publication of application: 04.01.2006
(73) Proprietor: Metris Therapeutics Limited, Reading RG6 1PT (GB)
(72) Inventor: KNOX, P.; c/o Metris Therapeutics Ltd, Reading RG6 1PT (GB)
(74) Representative: D'Arcy, Julia
(86) International application number: PCT/GB2004/001311
(87) International publication number: WO 2004/084872

(56) References cited:
- WO-A-01/19309
- WO-A-01/80937
- US-A- 3 902 493

## Description

This invention concerns drug delivery by way of mesh sleeves for use with devices adapted for insertion into the vagina, rectum, nasal or buccal cavity. The resulting apparatus exploit the highly vascularised nature of the tissue of bodily cavities such as the vagina, nose, rectum and mouth to deliver pharmaceutical agents to localised areas and/or into underlying tissues.

The vagina, rectum and nasal cavity provide an excellent route for the administration of Pharmaceutical agents due to the copious blood supply in these regions. Delivery of the agent through the tissue wall is generally fast. Any one of vaginal, rectal and nasal administration can be a preferred route when the drug can be destroyed by local conditions such as those encountered in the stomach. Other very important situations when the oral route is precluded are when vomiting has occurred, or is likely to occur, when the patient is unable to swallow successfully and when the patient is unconscious.

The vaginal delivery route is known to be useful for the delivery of pharmaceutical agents which have their site of action within tissues or organs close to the vagina, in particular, for administration to the vaginal tissues, uterus, ovaries and Fallopian tubes and other tissues and organs within the peritoneal cavity. Pathological lesions within and around these organs may also be treated in this manner.

The vagina may also be used for the delivery of pharmaceutical agents intended for use in other regions of the body. Administration through the vaginal cavity is particularly useful where other formulations are unsuitable, for example, in situations where pharmaceutical agents aggravate or upset the stomach, or are difficult or impossible to administer orally. Such agents may conveniently be administered intra-vaginally.

Intra-vaginal devices for the delivery of pharmaceutical agents and other materials into the vaginal cavity are known. Such devices are either of the type where a medicament is impregnated into the device, or of the type that carries an encapsulated medicament.

For example, U.S. Patent 4,309,997 discloses a moist, medicated vaginal tampon that is impregnated with a contraceptive agent and a medicament for the control of venereal disease.

U.S. Patent 4,318,405 discloses a tampon which has a capsule of disintegratable material partially embedded in one end. This tampon is inserted into the vagina and serves both to deliver and to retain the encapsulated medicament in the vaginal cavity. This patent also discloses a means for prewetting the tampon in order to activate the capsule.

U.S. Patent 5,527,534 discloses a sponge, impregnated with a liquid containing an effective amount of an active pharmaceutical agent, for insertion into the vaginal cavity.

U.S. Patent 5,273,521 discloses a tampon assembly that is adapted for carrying a medicament within a longitudinal bore formed within the tampon. The medicament can be selectively expelled into the vaginal cavity from the bore using a tubular inserter.

UK Patent GB 2364916 (METRIS THERAPEUTICS LIMITED) discloses a device adapted for insertion into the vagina, rectum or nasal cavity, wherein the device comprises a body which is preferably made from an absorbent material. A layer of fluid-impermeable material is present on at least part of said body and one or more pharmaceutical agents are disposed on the surface of said material remote from said body.

WO 01/80937-A discloses a drug delivery device for insertion in the vagina, rectum or nasal cavity comprising a drug such as fibrinolytic inhibitors. An elastic lattice comprising a drug is attached to the surface of the body of the device.

One disadvantage of the intra-vaginal devices described above is the device itself must be coated with pharmaceutical agent. Because the pharmaceutical agent necessarily has a limited shelf life, devices that are past the date by which they must be used must be discarded, so involving significant wastage of cost and resources. It is also necessary to manufacture different devices for different doses, and different drug regimens. Again, this is wasteful. Problems have also been encountered with consistency of production.

Moreover, requirements for the shape, size and structure of a particular intra-vaginal device vary among individuals according to a number of considerations such as the morphology of the individual concerned, the level of adsorption required and other considerations such as personal preferences. This is illustrated by the large variety of marketed tampons that are available. For example, the spectrum of tampons available in the marketplace extends from quite tough and rigid varieties at one extreme, which generally have a structured fluted surface, to the unstructured variety at the other extreme, which generally consist of a wad of cotton wool. Thus, in order to manufacture intra-vaginal drug delivery devices that are suitable for use by a wide range of individuals and individual preferences, it is necessary to adapt each different tampon accordingly for delivery of a pharmaceutical agent. This is a cumbersome process. Furthermore, since each device must be specifically tested for public use, separate (costly) clinical trials are required for each style of device.

The nasal and rectal mucosa and the buccal cavity also provide useful anatomical sites for systemic drug delivery. The nasal tissue is highly vascularized, providing an attractive site for rapid and efficient systemic absorption. The adult nasal cavity has a capacity of around 20 ml, with a large surface area (approximately 180 cm²) for drug absorption afforded by the microvilli present along the pseudostratified columnar epithelial cells of the nasal mucosa. For non-peptide small molecular compounds, intranasal bioavailability has been shown to be comparable to that of injections. The nasal mucosa has been shown to be amenable to the systemic absorption of certain peptides, as well as to nonpeptide drug molecules. The nasal route is advantageous for nonpeptide drugs that are poorly absorbed orally. One additional advantage to nasal absorption is that it avoids first-pass metabolism by the liver. The nasal route also offers advantages when rapid and regulated uptake of pharmaceutical agent is required, such as in the control of acute inflammation, acute respiratory disturbance, emesis, migraine and acute cardiological events.

Similarly, the buccal cavity can be used for systemic delivery of drugs. For example, oral surgery, such as periodontal surgery, can involve significant blood loss. Even in the case of tooth extractions, it can be difficult to control haemostasis. One method of reducing blood loss by speeding the blood clotting process is to use inhibitors of fibrinolysis. Most dental and medical text books suggest the use of tranexamic acid to achieve this. A prophylactic intravenous dose of tranexamic acid is known to reduce blood loss and there is evidence that topical application can also markedly reduce bleeding (for example, see Dunn, C. J. & Goa, K. L. (1999) Drugs, 57, 1005-1032 for review of the subject). It would thus be useful to have a device suitable for use during dental surgery, which is capable both of reducing blood loss by administration of a suitable pharmaceutical agent and of absorbing blood itself.

The present invention aims to solve these problems by providing an efficient method of adapting a wide variety of intra-vaginal, intra-rectal, intra-nasal and intra-buccal devices so that they form part of an apparatus, suitable for drug delivery. The present invention aims to provide an apparatus that is simple and comfortable to use, which is easier to manufacture than prior art devices and which leads to a more flexible approach to patient treatment.

### Summary of the invention

According to the present invention there is provided a mesh sleeve adapted for use with a device suitable for insertion into a bodily cavity and prepared separately from said device such that in use the sleeve envelopes the device, and wherein the sleeve comprises a pharmaceutical agent disposed thereon.

By "bodily cavity" is meant the vaginal, rectal, nasal or buccal cavities. Prior art intra-vaginal, intra-rectal and intra-nasal devices are manufactured and supplied as integral devices for particular therapeutic purposes. According to the present invention, mesh sleeve are prepared separately from the intra-cavity device. The sleeve of the invention can simply be placed over such a device prior to the insertion of the device into the individual to be treated. Assembly may either be directly after manufacture, by a health worker, by the individual patient concerned or by any other suitable person. Thus, mesh sleeves containing the relevant dose of drug(s) for a particular patient can be matched with an appropriate device depending upon the individual requirements of the patient to be treated. A fundamental advantage, particularly in the case of intra-vaginal devices, is that a particular mesh sleeve can be fitted over any suitable device, such as a tampon. The invention thus facilitates the generation of a bespoke apparatus for treating patients via these routes.

Furthermore, separating the production of the pharmaceutical agent (on the mesh sleeve) from the production of the device itself greatly simplifies the manufacturing process. Further, the small size of the sleeves relative to the device as a whole enables the space that is required for storage of the mesh sleeves to be kept to a minimum. This is particularly beneficial when one or more of the pharmaceutical agents to be applied requires specialist storage conditions. Travelling is also facilitated, since when away from home a patient might only need to carry a pack of mesh sleeves which could be used with tampon devices purchased locally.

As used herein, the term "device" refers to the part of the intra-vaginal, intra-rectal or intra-nasal device over which the mesh sleeve is placed.

The mesh sleeve envelopes the body of the device such that the device is inserted into the mesh sleeve. By "envelopes" is meant that the sleeve fits over and around the body of the device. However, the mesh sleeve need not completely envelope the body of the device and may in fact only fit over a portion of the full length of the device. In one embodiment, the mesh sleeve is a tubular structure, which has two open ends and fits over the body of the device like a tubular jacket. In a preferred embodiment, the sleeve forms a tubular structure having one substantially closed end and one open end, which thus fits over the device like a sock.

For example, where an intra-vaginal device is used, the mesh sleeve may have one closed end and one open end or may have one substantially closed end and one open end. By "substantially closed" is meant that the end of the mesh sleeve is closed to define an aperture that is smaller than the bore of the device around which it is to fit. The sleeve thus need not be completely closed at this end as long as it prevents the device from passing through it.

For dental applications, such as where an intra-buccal cavity device is used, the mesh sleeve may have either two open ends or may have one closed end and one open end.

The mesh sleeve should not constrict the expansion of the device when in use and should be able to spread with the device when the body of the device expands due to the absorption of bodily fluid during use. This ensures that the mesh sleeve fits closely with the body of the device during use, such that the mesh sleeve is held securely in place. This secure fit also ensures that the mesh sleeve is removed simultaneously with the device when the apparatus is removed from a patient. As used herein, the term 'apparatus' is used to mean an intra-vaginal, intra-rectal, intra-nasal or intra-buccal device adapted for insertion into the vagina, rectum, nasal or buccal cavity respectively, wherein the body of the device is enveloped by a mesh sleeve according to the present invention.

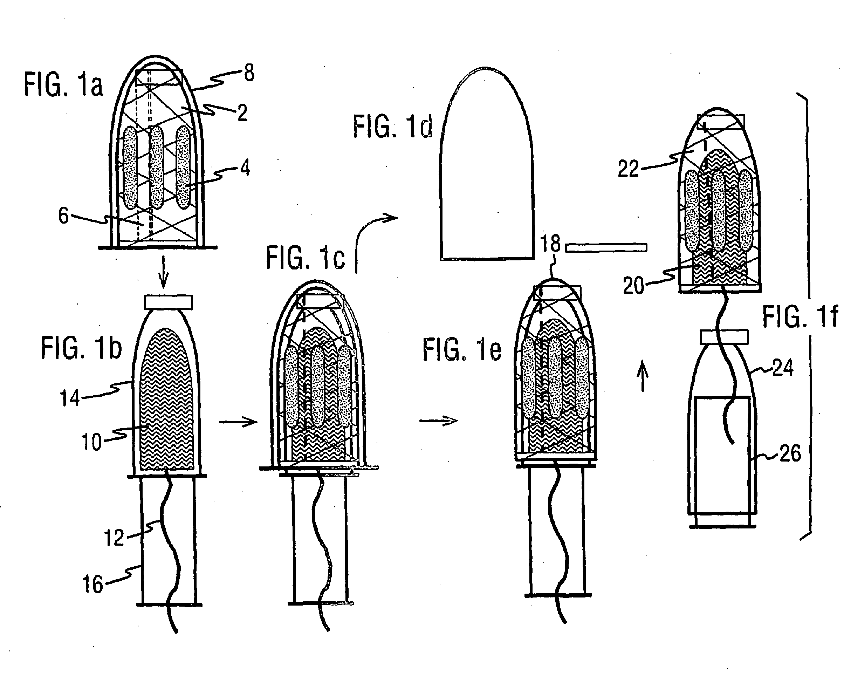

The sleeve should remain substantially intact and remain attached to the body of the device throughout the period of use and removal of the apparatus. In a preferred embodiment, this ability to expand is in part due to the presence of an overlap or fold in the mesh sleeve (see Figure 1a (6)), which accommodates the expansion of the mesh sleeve around the body of the device. This overlap could be produced by spot-welding (i.e. loosely tacked down) in a manner which enables the bonds to break when expansion occurs. In a further embodiment, the ability to expand is in part a property of the mesh design, in that the openings in the mesh get bigger as the sleeve stretches. The ability to expand may also be a result of the inherent elasticity of the material from which the mesh sleeve is made. Of course, the ability to expand may be due to a combination of one or more of the following: the presence of an overlap in the mesh sleeve, the elasticity of the mesh material and the properties of the mesh design.

In a further embodiment, the sleeve may be attached to one or more "tethering" components that facilitate the convenient placement of this sleeve over and around the body of the device. This tether is purely for the purpose of fabrication and fulfils no pharmaceutical function. The tether may pass a small distance into the body of the device or alternatively can span one axis of the body of the device.

The mesh sleeve can be made of a variety of different materials, as the skilled reader will appreciate. The material should of course be suitable for use inside body cavities and thus should be chemically inert, insoluble and non-immunogenic, as well as having smooth surfaces to ensure that it is comfortable in use. In addition, the material should be pliable to allow the sleeve to fit smoothly over any irregularities in the shape of the device. The material may also have a degree of elasticity to ensure that the sleeve fits closely over the device and cannot be easily dislodged from its enveloping position. In a preferred embodiment, the mesh is a mesh material formed from cotton. The cotton may be a non-wettable cotton. In an alternative embodiment, the mesh is a mesh material formed from a synthetic material such as viscose, Cotton Lycra® and so on.

The pharmaceutical agent(s) may be disposed on the mesh sleeve in a number of different ways. For example, the pharmaceutical agent may be adsorbed onto or into the structure of the mesh itself. Alternatively, the pharmaceutical agent may be coated onto the mesh sleeve. This is preferable in situations where the pharmaceutical agent is highly reactive. In the embodiment in which a pharmaceutical agent is coated onto the mesh sleeve, the mesh itself can form a fluid impermeable layer. Where the mesh itself forms a fluid impermeable layer, the material from which the mesh is made is preferably a non-wettable cotton. The presence of a fluid-impermeable layer prevents or reduces the absorption of the pharmaceutical agent into the body of the device.

The distribution of pharmaceutical agent may be homogenous, covering the majority or all of the mesh, or may be heterogenous, occurring in discrete areas of concentration interspersed across the mesh sleeve. Ideally, the pharmaceutical agent should be arranged such that in use the epithelia of the relevant body cavity is not exposed to areas of high concentration of pharmaceutical agent; it is thus preferable for the pharmaceutical agent to be spread equally over the sleeve.

In a still further embodiment, the pharmaceutical agent may be affixed to the mesh sleeve in the form of discrete patches or coupons. The term 'coupons' is used herein to describe a discrete and separate measure of pharmaceutical agent formed with a suitable excipient and/or carrier into a capsule that can be attached to the mesh sleeve. In a preferred embodiment, a pharmaceutical agent is coated onto a layer of two-sided pharmaceutical tape, or some equivalent material. This pharmaceutical tape acts as a fluid-impermeable layer and reduces or prevents the pharmaceutical agent from being absorbed into the body of the device itself. This can be a significant advantage, particularly since it means that the dosage of pharmaceutical agent contained on the mesh is delivered to the epithelia and thus the patient rather than being absorbed into the tampon. More precise dosage delivery is thus achieved, irrespective of the quantity of menstrual fluid that is being passed. To generate such a coated tape, the pharmaceutical agent may be prepared as a slurry and dried onto the tape before being scored in the required manner in order to make a 'coupon' comprising a section of two-sided pharmaceutical tape having a pharmaceutical agent spread on one side of it. The coupon may then be fixed onto the mesh sleeve by the sticky side of the pharmaceutical tape to which no pharmaceutical agent is coated. Preferably, the pharmaceutical agent(s)/coupons are disposed on/attached to the material of the mesh sleeve and should be in place prior to the placement of the mesh sleeve over the device. Alternatively, the mesh sleeve can be placed over the body of the device in a first stage and the pharmaceutical agent/coupon disposed on/attached in a second stage.

There may be just one or alternatively a number of coupons attached to the mesh sleeve. For example, there may be 1, 2, 3, 4, 6, 8, 10, 20, 50, 100 or more such coupons attached to the mesh sleeve. The number of coupons attached to the mesh sleeve may vary depending on the pharmaceutical agent, the dosage requirements of the particular patient to be treated or because of other considerations. For example, undesirable responses of vaginal wall tissue to irritant pharmaceutical agents may be reduced by using a greater number of coupons each containing a smaller amount of the pharmaceutical agent, thereby reducing the exposure of any one point on the vaginal wall to the agent. Furthermore, where necessary, bodily absorption of the pharmaceutical agent can be increased by spreading the required dosage of pharmaceutical agent over a large number of coupons and thus increasing the area for absorption rather than having the same amount of pharmaceutical agent shared between a smaller number of coupons.

The shape of these coupons may be regular or irregular. Conveniently, the coupons are in the form of rectangles, circles, squares, triangles, ellipses or circumferential rings. Where there is more than one coupon, the coupons may have the same or different dimensions.

The total amount of pharmaceutical agent contained on each mesh sleeve will vary depending on the activity of the agent and the eventual tissue concentration to be attained. Generally, the amount will be between 100 µg to 1 g, preferably between 100 µg and 100 mg. The amount selected will depend, of course, on the dosage prescribed, but undesirably high dosages may be avoided using the mesh sleeve of the invention as the concentration in the vicinity of the tissue wall is maintained at a high level due to the structure of the body of the device upon which it is mounted.

The nature of the pharmaceutical agent used on the mesh sleeve will depend upon the requirements of the individual to whom the therapy is being administered. The amount of pharmaceutical agent in a coupon may vary depending upon the dosage requirements of the particular individual to be treated or because of other considerations, such as absorption considerations as mentioned above. Preferably, pharmaceutical agents will be low molecular weight entities with molecular weights less than 1,500 Daltons but ideally below 700 Daltons. Pharmaceutical agents may also be peptides or proteins with biological activity.

Preferably, the pharmaceutical agent in the coupon is in conjunction with an appropriate pharmaceutical carrier. Suitable pharmaceutical carriers include large, slowly metabolised macromolecules such as proteins, polysaccharides, polylactic acids, polyglycolic acids, polymeric amino acids, cellulose-type polymers and amino acid copolymers. In addition to the pharmaceutical agent, the coupon may alternatively or additionally comprise a pharmaceutical excipient. The excipients used in the formulation of the pharmaceutical layer may remain essentially unaltered during the period of use of the device.

Alternatively, the excipients may dissolve and/or diffuse away from the layer of pharmaceutical agent. Where a wetting agent is required in order to coat the double sided pharmaceutical tape, a wetting agent may also be present in the coupon. The pharmaceutical agent may be formulated with a penetration enhancer.

The mesh sleeve of the invention may be used in conjunction with a number of different devices. For example, the body of the device may comprise absorbent or non-absorbent material. In the case of an intra-vaginal device, the body of the device preferably comprises absorbent material. As will be known to the skilled reader, the absorbent material may be a cellulose or cellulose derivative fibre, cotton, starch, rayon, sponge, woodpulp, polyolefin, polyester, polyamide, polyurethane, cross-linked carboxymethylcellulose, acrylic acid, methacrylic acid, 2-acrylamido-2-methyl propane sulphonic acid or any mixture of the above. An alternative material can be derived from the formation of hydrogels by polymerization of agents such as unsaturated acid-containing monomers. It should be recognized that some materials are inherently absorbent while in other cases it is the mass of fibres rather than the individual fibre which is absorbent. The addition of surfactants to the surface of fibres can considerably enhance absorbent properties. Suitable surfactants could be anionic or non-ionic in nature.

For an intra-vaginal device, the presence of absorbent material allows the device to fulfil the function of a conventional tampon in addition to its role in delivering pharmaceutical agents. Accordingly, it may be used during menstruation in place of a conventional absorbent article. Such a device may comprise essentially a normal tampon modified so that the mesh sleeve can fit over it. A tampon as described in UK Patent GB 2364916 may be used in conjunction with the mesh sleeve of the present invention.

In order to perform the dual function of delivery of pharmaceutical agent and absorption of bodily fluid, the body of the device preferably has an absorbency between 0.6 and 1.35 ml per cm³ of the body of the device, more preferably between 0.75 and 1.2 ml per cm³ of the device. The absorbency of the body of the device may be measured by applying water to one end of a suspended device and determining the maximum uptake of fluid without leakage. As such, the absorbency may be defined as the volume of water that can be applied at room temperature at a rate of 1 ml per 10 seconds.

During menstruation, such an intra-vaginal device may thus be used to deliver the pharmaceutical agent and to function simultaneously as a conventional absorbent article. Where the apparatus is intended for use on days on which there is menstruation, or where the onset of menstruation is anticipated, a pharmaceutical agent formulated for more immediate release and uptake through the vaginal mucosa, for example in the form of a dry powder, may be used. Fast delivery of the complete dosage of pharmaceutical agent is preferable in order that the apparatus may be replaced after the recommended period for replacement of such articles, which is generally between 4 and 8 hours. Furthermore, if menstrual flow is particularly heavy and, for example in order to prevent leakage of menses, it becomes necessary to replace the apparatus sooner than expected, then it is essential that complete delivery of the pharmaceutical agent will have occurred prior to removal of the apparatus. In this manner, delivery of an incorrect dosage will be avoided. The apparatus comprising an intra-vaginal device may be used at any stage of the menstrual cycle. The apparatus comprising intra-vaginal devices of the present invention may be used intermittently or cyclically.

It is expected that a plurality of absorbent articles will be used during any one day while menstruation occurs. Use of the present invention may be complemented by use of conventional absorbent products. For example, the apparatus of the present invention may be used alternately with conventional absorbent articles during a period of 24 hours.

Preferably, the device is shaped in such a way that on insertion into the vaginal, rectal or nasal cavity, the physical interaction between the pharmaceutical agent and the mucosal tissue is maximised. In addition, the shape of the device should cause minimal discomfort to the wearer. As the skilled reader will appreciate, the intra-vaginal or intra-rectal device may also be substantially cylindrical, substantially spherical or substantially ellipsoid. The intra-nasal device of the invention should be in the form of a hollow tube adapted for insertion into a nostril. In this manner, the pharmaceutical agent may be delivered, while still allowing the patient to breathe comfortably. The intra-buccal device according to the present invention is preferably 2-3 cm long with a diameter of 0.75-0.85 cm, such as is standard for absorbent pads used in dental procedures.

For an intra-buccal device, absorbent material is preferably used, for example, to allow the device to fulfil the function of a conventional intra-buccal pad that is used to absorb blood and stop bleeding during dental operations. In order to perform the dual function of delivery of pharmaceutical agent and absorption of bodily fluid such as blood, the body of the device preferably has an absorbency between 0.6 and 1.35 ml per cm³ of the body of the device, more preferably between 0.75 and 1.2 ml per cm³ of the device.

According to an alternative embodiment of the invention, the body of the device of the invention does not comprise an absorbent material.

Conveniently, the intra-vaginal apparatus for use on non-menstruation days comprises pharmaceutical agent in a sustained-release formulation. The apparatus may thus be worn for an extended period of time of up to 12 hours, preferably up to 8 hours, avoiding the need for regular replacement.

The mesh sleeve of the invention may be used in therapy. For example, in conjunction with an intra-vaginal, intra-rectal, intra-nasal or intra-buccal device, it may be used to deliver pharmaceuticals for the treatment of a range of clinical conditions, including, but not limited, to cardiovascular disease, respiratory disease, infective disease, metabolic disease, diseases of the central and peripheral nervous system, diseases of the urogenital tract, psychiatric conditions, gastrointestinal disorders, disorders of the special senses, endocrinological disorders, muscoskeletal disorders, bleeding and any disease that can be treated by drugs administered via other routes including oral, intravenous, intramuscular, subcutaneous and some topical routes. In a preferred embodiment of the invention, pharmaceutical agents delivered using the present invention may be anti-inflammatories and haemostasis-modifying agents.

The present invention may also be used for the delivery of pharmaceutical agents for the treatment of multiple symptoms. This may be achieved by the administration of more than one class of pharmaceutical agent using separate sleeves on devices inserted within a short time of one another and where the time interval is not less than ten minutes. Alternatively the mesh sleeve may be manufactured such that one sleeve comprises more than one pharmaceutical agent. One particularly preferred combination of pharmaceutical agents for delivery using a mesh sleeve of the present invention on an intra-vaginal device is the combination of an anti-inflammatory prostaglandin synthesis inhibitor and a fibrinolytic inhibitor.

For the intra-vaginal device mesh sleeve, the pharmaceutical agent may be one for use in the management of female-specific disorders, for example in conditions associated with the menstrual cycle including dysmenorrhea, endometriosis, fibroids and heavy menstrual bleeding.

For the intra-buccal device, in addition to the delivery of pharmaceutical agents systemically, specific pharmaceutical agents might be used, for example to reduce blood loss during periodontal surgery using fibrinolysis inhibitors.

Pharmaceutical agents that act systemically may also be administered with a mesh sleeve of the present invention. For example, used with an intra-vaginal device, the present invention may also be used to deliver other agents through the vaginal mucosa, such as dietary supplements, including vitamins and metal ions, which may be difficult or inconvenient to administer via other routes. Thus, the terms 'treatment' and 'therapy' as used herein also encompass the delivery of dietary supplements to an individual.

In one embodiment, the pharmaceutical agent may be a steroid, preferably a sex steroid, glucocorticoid or mineralocorticoid. The oestrogenic and prostagenic steroids can bring about a number of therapeutic benefits for clinical syndromes associated with the female cycle. The mesh sleeve of the present invention is suitable for delivery of such steroids when the treatment period is not continuous. Thus steroids can be administered using a mesh sleeve and intra-vaginal device for up to a maximum of six days each month.

The pharmaceutical agent used according to the present invention may modulate thrombotic and fibrinolytic cascades and thus control the abnormal bleeding that may occur during menstruation. Pharmaceutical agents that inhibit fibrinolysis when taken orally are known to be effective in the control of menstrual bleeding. These fibrinolytic inhibitors work by inhibiting the breakdown of blood clots in the spiral arteries and arterioles. Fibrinolytic inhibitors have been reported to decrease blood loss by up to 50% and their efficacy is greatest in those patients with heavy blood loss. However, there is concern that oral administration of anti-fibrinolytic agents might be associated with systemic thrombotic complications. In particular, to achieve a therapeutically useful systemic concentration through oral administration, it is usually necessary to administer high levels of the pharmaceutical agent and these high levels may cause side effects. In contrast, the device of the present invention enables the pharmaceutical agent to be administered close to the treatment site, thus lowering the systemic concentration and reducing the occurrence of unwanted side-effects. In addition to use with intra-vaginal devices, a fibrinolytic inhibitor is a particularly preferred pharmaceutical agent for delivery using apparatus comprising an intra-buccal device.

Anti-fibrinolytic agents suitable for administration using the present invention include tranexamic acid, acexamic acid, aminocaproic acid, aprotinin and ethamsylate. Certain biologically active peptides and proteins, for example the snake venom enzyme Batroxobin, may also be used.

Other preferred pharmaceutical agents for delivery using intra-vaginal devices with mesh sleeves of the present invention include anti-inflammatory agents, that are known to reduce the symptoms associated with painful menstruation. Such agents may be steroidal or non-steroidal. Examples of non-steroidal and-inflammatory agents (NSAIDs) include prostaglandin synthesis inhibitors and prostaglandin receptor antagonists, and in addition to providing pain relief may also alter blood clotting. NSAIDs may be used in the treatment of Dysfunctional Uterine Bleeding (DUB) and in the treatment of dysmenorrhoea.

One other preferred class of anti-inflammatories for use in the invention are cyclooxygenase inhibitors, for example acetylsalicylic acid, salicylsalicylic acid, salicylic acid, trilisate, disalcid and salts thereof.

Further preferred anti-inflammatory pharmaceutical agents include diclofenac, flurbiprophen, naproxen, piroxicam, mefenamic acid, indomethacin, sulindac, meclofenamate, diflunisal, tolmetin, acetominophen, ibuprofen, oxaprozin, etodolac, fenoprofen, ketoprofen and nabumetone.

Tocolytics form another class of pharmaceutical agents useful for delivery using the apparatus of this aspect of the invention. This class of compounds relax the muscle wall of the uterus and may relieve painful menstruation and may be used in the treatment of "cramps" associated with menstruation. Tocolytics include anti-muscarinic pharmaceutical agents and β₂-agonists. Preferred anti-muscarinic agents include atropine sulphate (or its other salts), benztropin mesylate, biperiden lactate (or its other salts), cyclopentolate hydrochloride, dicyclomine hydrochloride, enepromium salts, glycopyrronium bromide, homatropin methobromide, hyoscine butylbromide (or its other salts), ipratropium bromide, propantheline bromide, alverine citrate or mebeverine hydrochloride. Preferred β₂-agonists include short-acting agents, such as ritrodine, orciprenaline, salbutamol, terbutaline, and long-acting selective agents, such as salmeterol or formoterol. Other pharmaceutical agents which have a tocolytic effect, and which may be delivered using the apparatus of the present invention, include aminophylline, which brings about a tocolytic effect by inhibiting the enzyme phosphodiesterase, and calcium antagonists such as nifedipine.

Mesh sleeves adapted for use with intra-rectal, intra-nasal and intra-buccal devices may equally comprise any of the above-mentioned pharmaceutical agents. In addition, further pharmaceutical agents useful for delivery in this manner include adrenaline, sodium nitroprusside, an anti-emetic, such as ondansetron, an anti-migraine, such as sumatriptan, a bronchodilator such as salbutamol or theophylline, or a diuretic such as frusemide.

Any of the above-mentioned pharmaceutical agents may be in the form of a mixture of optical isomers. Alternatively, the pharmaceutical agent may be enantiomerically pure. Furthermore, as and when new pharmaceutical agents are developed these will also be suitable for incorporation into a mesh sleeve according to this invention.

In use, a mesh sleeve of the invention is mounted on a suitable device and together they are inserted into the vaginal, rectal, nasal or buccal cavity where they are retained for a period of time between 30 minutes and 12 hours, preferably between 4 and 8 hours. During this period, the surface of the apparatus remains in a position, contacting the vaginal wall. Molecules of the pharmaceutical agent diffuse through the mucosal tissue into the underlying tissues, blood and lymph. Accordingly, a further aspect of the invention provides an intra-vaginal, intra-rectal, intra-nasal or intra-buccal apparatus comprising a mesh sleeve according to any one of the aspects of the invention described above, and a device adapted for insertion into the vagina, rectum or nasal cavity, respectively.

The mesh sleeve of the invention may thus be used in a method of treating a disease in a patient, by administering a pharmaceutical agent to the patient using the mechanism described in detail above.

Generally, a device to be used with a mesh sleeve according to the invention comprises withdrawal means. For example, in the case of the intra-vaginal or intra-rectal device, the withdrawal means is a string attached to the body of the device that may be pulled to remove the device from the vagina or rectum.

The device may also comprise insertion means. Preferably the insertion means comprises a first hollow cylindrical tube defining a cartridge for receiving the device body and a second hollow cylindrical plunger slidably received within said first cylindrical tube.

A backing may be attached to the mesh sleeve. The backing serves a protective function and is removed when the mesh sleeve is in use. The backing may be made from a rigid material such as cardboard or plastic, or can form an easily-peelable protective layer. In a preferred embodiment, the backing is in the form of a cone-shape that envelopes the mesh sleeve. In addition to serving a protective function, the presence of the backing may provide resistance, which allows pressure to be applied to the mesh sleeve thus facilitating the coating of a pharmaceutical agent onto the mesh or facilitating the attachment of coupons onto the mesh sleeve. The presence of the backing also facilitates the handling of the mesh sleeve prior to use.

The mesh sleeves, with or without backing, and the intra-cavity device may be contained in separate sterile packages prior to use. Alternatively, a combined apparatus comprising the device with the mesh sleeve already fitted may be contained in a single sterile package prior to use. A number of sterile packages containing separate mesh sleeves, and/or devices may be packaged together, for example in a box. A pharmacist might dispense to a patient a box containing a plurality of sterile packages comprising mesh sleeves according to the invention, along with a series of intra-vaginal, intra-rectal, intra-nasal or intra-buccal devices for a course of prescription.

Specific embodiments of the present invention are now described, with reference to the accompanying drawings in which:
Figure 1 shows a mesh sleeve and an intra-vaginal device and how these fit together.

### Example 1

With reference to Figure 1a, one embodiment of the present invention provides a mesh sleeve (2) to which are attached one or more coupons (4) comprising one or more pharmaceutical agents. The presence of an overlap (6) in the mesh material enables the mesh sleeve to expand in use. The mesh sleeve is attached to a backing (8).

Figure 1b shows one embodiment of an intra-vaginal or intra-rectal device (10). In use, the body of the device (11) may absorb fluid, causing the body of the device to swell. A piece of string (12) is attached to the body of the device, which preferably remains outside the individual's body when in use. This string enables the device to be removed from the patient by pulling the string. The embodiment of the invention in Figure 1b shows an inserting means comprising a first hollow cylindrical tube (14) defining a cartridge for receiving said device and a second hollow cylindrical plunger (16) slidably received within said first cylindrical tube.

The body of the device is inserted into the mesh sleeve and then the backing is removed. This enables the apparatus to be assembled without the pharmaceutical layer being touched. Figure 1c shows the mesh sleeve together with the backing of Figure 1a enveloping the body of the intra-vaginal or intra-rectal device of Figure 1b. The removed backing is shown in Figure 1d and the intra-vaginal or intra-rectal device with the mesh sleeve enveloping it once the backing has been removed is shown in Figure 1e.

In order to insert the combined sleeve + device into the individual to be treated, the apparatus, as shown in Figure le, is inserted into the patient's vagina or rectum in a tip (18) first orientation such that the first hollow cylindrical tube (14) is preferably within the individual's vagina or rectum whilst the second hollow cylindrical plunger (16) preferably remains outside. The second hollow cylindrical plunder is then plunged inside the first hollow cylindrical tube such that the body of the intra-vaginal or intra-rectal device exits through the distal end of the first tube, catching the mesh sleeve as it exits such that the body of the intra-vaginal or intra-rectal device (20) with the mesh sleeve (22) enveloping it are placed further within the individual's body. The first hollow cylindrical tube (24) together with the second hollow cylindrical plunger (26) inside it are then pulled out from the individual's body (Figure 1f).

### Example 2

Devices were constructed manually in order to demonstrate function. In this example, two intra-vaginal devices are compared; first, a tampon with patches of pharmaceutical struck directly onto the tampon, and second, a tampon enveloped with a mesh sleeve device of the invention, with patches of pharmaceutical stuck onto the mesh sleeve device rather than the tampon directly.

A layer of pharmaceutical and excipients was first cast onto double-sided pharmaceutical tape. After drying to completeness the tape was cut into identically sized 'coupons'. Eight of these coupons were applied to either a tampon or to the mesh sleeve device of the invention. The sleeve was held in place on the surface of a plastic mould which was identical in shape and size to the tampons. Coupons were fixed to tampons or sleeves by removing the protective layer from the second side of the tape and using gentle compression for around one minute.

After storage for twenty four hours at ambient temperature, the sleeves were transferred to the surface of tampons.

The final intra-vaginal devices, namely coupons fixed directly to tampons or coupons fixed to tampons via the sleeve were then compared for their ability to release an active pharmaceutical, in this case mefenamic acid, when placed in an aqueous environment.
This enabled functional 'equivalence' of the devices to be confirmed.

For commercial production, all of the procedures will require complete automation. *Construction of mesh sleeve*

Gauze bandage material was cut into lengths and placed with a single pleat over a 30cm length of rigid tubing the diameter of a regular sized tampon. Elastic bands were used to keep the material in place at each end with one elastic band in the middle. A soldering iron was used to 'spot' weld together the edges of the gauze.

The tube of gauze was carefully removed and cut into lengths 5mm longer than that of a regular tampon. Each of these cut lengths was placed over a plastic mould identical in shape and size to a tampon and at the tapered end spot welding used to seal the end. The blank sleeves were maintained in this format until application of coupons.

### Preparation of coupons

### A slurry of pharmaceutical was formed at room temperature.

50gm of mefenamic acid (2-[2,3-Dimethylphenylamino] benzoid acid was suspended in a mixture of 100gm of water and 100gm of ethanol. This was stirred at low speed for thirty seconds. To this was added 45gm hydroxyphenylmethylcellulose and the mixture stirred for a further 20 seconds. Following the addition of 5 gm polysorbate (Tween 80) the suspension was stirred at high speed for 30 seconds and then for four hours at low speed.

Double sided medical tape (cat. No 9877; 3M Medical Specialities) is supplied with one sticky side and one side covered in protective film. The slurry of mefenamic acid was layered with a glass spreader onto the sticky side to achieve a final dried thickness for the laminated coupon of 0.2mm.

Individual coupons were cut from the laminated layer with the dimensions of 35mm long and 3.5mm wide. Coupons were stored for between one and three days and then after removing the protective backing layer eight coupons per tampon were applied longitudinally between the flutes of the tampons or applied at regular spacing intervals to the mesh sleeves. The total amount of mefenamic acid per tampon or per sleeve was 80mg.

### Equivalence of devices

Intravaginal devices constructed by the direct application of coupons to tampons were compared to intravaginal devices constructed using the mesh sleeve.

Devices were placed in beakers containing 100ml water and stirred gently for varying periods of time. The fluid phase was removed brought to a pH of 3.0 with sodium hydroxide and the concentration of mefenamic determined by spectrophotometry.

In a typical result using six of each type of device, the mefenamic acid recovered in the aqueous phase was 72.8mg (mean, standard deviation 3.6mg) for devices with directly attached coupons and 73.7mg (mean, standard deviation 6.2mg) for devices constructed by attaching coupons via the sleeve.

In this simple *in vitro* evaluation, both sets of devices behaved effectively identically.

### Clinical evaluation of devices

The purpose of the device described here is to administer mefenamic acid via the intravaginal route. In one clinical study, intravaginal devices, using the direct process of attachment of coupons to tampons were prepared to cGMP (Good Manufacturing Procedure); devices were prepared containing 20, 40 or 80 mg per tampon.

These were administered to human volunteers in a study conforming to Good Clinical Procedure and circulating plasma concentrations of mefenamic acid were determined after drawing blood samples. The results are shown below.

| Total mefenamic acid per tampon | No. of volunteers | Max. plasma conc. (ng/ml) | Standard deviation | Accumulated dose (0-7) |
|---|---|---|---|---|
| 20 | 6 | 4.23 | 1.73 | 44.7 |
| 40 | 6 | 14.9 | 6.3 | 197 |
| 80 | 6 | 19.5 | 5.9 | 257 |

These results indicate that the devices can be used to administer mefenamic acid intravaginally in humans.

## Claims

1. A mesh sleeve adapted for use with a device suitable for insertion into a bodily cavity and prepared separately from said device such that in use the sleeve envelopes the body of the device, and wherein the sleeve comprises a pharmaceutical agent disposed thereon.

2. A sleeve according to claim 1, wherein the sleeve is adapted for use with a device suitable for insertion into the vaginal, rectal, nasal or buccal cavity.

3. A mesh sleeve according to claim 1 or claim 2, wherein said sleeve has one open end and one substantially closed end.

4. A mesh sleeve according to claim 1 or claim 2, which is open at both ends.

5. A mesh sleeve according to any one of claims 1 to 4, wherein said mesh sleeve can expand when the device expands during use.

6. A mesh sleeve according to claim 5, wherein the ability to expand is conferred by the presence of an overlap of mesh material.

7. A mesh sleeve according to claim 5, wherein the ability to expand is conferred by the elasticity of the mesh material.

8. A mesh sleeve according to claim 5, wherein the ability to expand is conferred by a combination of the elasticity of the mesh material and the presence of an overlap of mesh material.

9. A mesh sleeve according to any one of claims 1 to 8, which comprises a tethering component suitable for attachment of the sleeve to the body of the device.

10. A mesh sleeve according to any one of claims 1 to 9, wherein the material from which the mesh sleeve is made is cotton, such as non-wettable cotton.

11. A mesh sleeve according to any one of claims 1 to 10, which has 1, 2, 3, 4, 6, 8 10, 20, 50,100 or more discrete pharmaceutical coupons attached thereto.

12. A mesh sleeve according to any one of the preceding claims, wherein said pharmaceutical agent is formulated with one or more pharmaceutically-acceptable excipients and/or carriers.

13. A mesh sleeve according to claim 12, additionally comprising a wetting-agent.

14. A mesh sleeve according to any one of the preceding claims, wherein said pharmaceutical agent is in the form of a sustained release composition.

15. A mesh sleeve according to any one of the preceding claims, wherein the amount of pharmaceutical agent disposed on the surface is between 10 µg and 1 g.

16. A mesh sleeve according to any one of claims 1 to 15, wherein said one or more pharmaceutical agents are selected from one or more members of the group consisting of an anti-fibrinolytic agent, such as tranexamic acid or aminocaproic acid; an anti-inflammatory agent, such as ibuprophen or mefenamic acid; a tocolytic agent, such as hyoscine or ritrodine; a haemostasis-modifying agent; sex steroid; glucocorticoid; mineralocorticoid and dietary supplements.

17. A mesh sleeve according to any one of claims 1 to 16, wherein said device is an intra-nasal device or an intra-rectal device, and wherein said one or more pharmaceutical agents are selected from one or members of the group consisting of adrenaline, sodium nitroprusside, anti-emetics, such as ondansetron, anti-migraines, such as sumatriptan, a bronchodilator such as salbutamol or theophylline, or diuretics such as frusemide.

18. A mesh sleeve according to any one of claims 1 to 17, wherein said mesh sleeve is attached to backing.

19. An intra-vaginal, intra-rectal, intra-nasal or intra-buccal apparatus comprising a mesh sleeve according to any one of the preceding claims, and a device adapted for insertion into the vagina, rectum, nasal or buccal cavity, respectively.

20. An apparatus according to claim 19, wherein the body of the device is made from an absorbent material.

21. An apparatus according to claim 20, wherein said absorbent material is cellulose or cellulose derivative fibres, cotton, starch, rayon, sponge, woodpulp, polyolefin, polyester, polyamide, polyurethane, cross-linked carboxymethylcellulose, acrylic acid, methacrylic acid, 2-acrylamido-2-methyl propane sulphonic acid or a mixture thereof, or a hydrogel.

22. An apparatus according to any one of claims 19 to 21, wherein said device is a tampon.

23. An apparatus According to any one of claims 19 to 22 further comprising inserting means.

24. An apparatus according to claim 23, wherein said inserting means comprises a first hollow cylindrical tube defining a cartridge for receiving said device and a second hollow cylindrical plunger slidably received within said first cylindrical tube.

25. The mesh sleeve according to any one of claims 1 to 18 or apparatus according to any one of claims 19-24 for use in therapy.

## Patentansprüche

1. Eine Netzhülse, die zur Verwendung mit einem Hilfsmittel, das zur Einführung in eine Körperöffnung geeignet ist, angepasst ist und separat von dem Hilfsmittel präpariert wird, so dass die Hülse bei Verwendung den Körper des Hilfsmittels einhüllt, und wobei die Hülse einen darauf verteilten pharmazeutischen Wirkstoff beinhaltet.

2. Hülse gemäß Anspruch 1, wobei die Hülse zur Verwendung mit einem Hilfsmittel, das zur Einführung in die vaginale, rektale, nasale oder bukkale Öffnung geeignet ist, angepasst ist.

3. Netzhülse gemäß Anspruch 1 oder Anspruch 2, wobei die Hülse ein offenes Ende und ein im Wesentlichen geschlossenes Ende aufweist.

4. Netzhülse gemäß Anspruch 1 oder Anspruch 2, die an beiden Enden offen ist.

5. Netzhülse gemäß einem der Ansprüche 1 bis 4, wobei sich die Netzhülse weiten kann, wenn sich das Hilfsmittel während der Verwendung weitet.

6. Netzhülse gemäß Anspruch 5, wobei die Fähigkeit zum Weiten durch das Vorhandensein einer Überlagerung des Netzmaterials verliehen wird.

7. Netzhülse gemäß Anspruch 5, wobei die Fähigkeit zum Weiten durch die Elastizität des Netzmaterials verliehen wird.

8. Netzhülse gemäß Anspruch 5, wobei die Fähigkeit zum Weiten durch eine Kombination der Elastizität des Netzmaterials mit dem Vorhandensein einer Überlagerung des Netzmaterials verliehen wird.

9. Netzhülse gemäß einem der Ansprüche 1 bis 8, das eine Anbindungskomponente beinhaltet, die zur Anbringung der Hülse an dem Körper des Hilfsmittels geeignet ist.

10. Netzhülse gemäß einem der Ansprüche 1 bis 9, wobei das Material, aus dem die Netzhülse hergestellt wird, Baumwolle wie etwa nicht benetzbare Baumwolle ist.

11. Netzhülse gemäß einem der Ansprüche 1 bis 10, die 1, 2, 3, 4, 6, 8, 10, 20, 50, 100 oder mehr daran angebrachte einzelne pharmazeutische Coupons aufweist.

12. Netzhülse gemäß einem der vorhergehenden Ansprüche, wobei der pharmazeutische Wirkstoff mit einem oder mehreren pharmazeutisch akzeptablen Vehikeln und/oder Trägern formuliert wird.

13. Netzhülse gemäß Anspruch 12, die zudem einen Benetzungswirkstoff beinhaltet.

14. Netzhülse gemäß einem der vorhergehenden Ansprüche, wobei der pharmazeutische Wirkstoff in der Form einer Zusammensetzung mit Langzeitfreigabe vorliegt.

15. Netzhülse gemäß einem der vorhergehenden Ansprüche, wobei die Menge an auf der Oberfläche verteiltem pharmazeutischem Wirkstoff zwischen 10 µg und 1 g beträgt.

16. Netzhülse gemäß einem der Ansprüche 1 bis 15, wobei der eine oder die mehreren pharmazeutischen Wirkstoffe aus einem oder mehreren Elementen aus der Gruppe, bestehend aus einem Antifibrinolytikum wie etwa Tranexamsäure oder Aminocapronsäure; einem Antiinflammatorikum wie etwa Ibuprofen oder Mefenaminsäure; einem Tokolytikum wie etwa Hyoscin oder Ritodrin; einem Hämostase modifizierenden Wirkstoff; einem Sexualhormon; einem Glucocorticoid; Mineralocorticoid- und Nahrungsergänzungsmitteln, ausgewählt werden.

17. Netzhülse gemäß einem der Ansprüche 1 bis 16, wobei das Hilfsmittel ein intranasales Hilfsmittel oder ein intrarektales Hilfsmittel ist, und wobei der eine oder die mehreren pharmazeutischen Wirkstoffe aus einem oder mehreren Elementen aus der Gruppe, bestehend aus Adrenalin, Natriumnitroprussid, Antiemetika wie etwa Ondansetron, Migränemittel wie etwa Sumatriptan, einem Bronchodilatator wie etwa Salbutamol oder Theophyllin oder Diuretika wie etwa Furosemid, ausgewählt werden.

18. Netzhülse gemäß einem der Ansprüche 1 bis 17, wobei die Netzhülse an einer Verstärkung angebracht wird.

19. Eine intravaginale, intrarektale, intranasale oder intrabukkale Vorrichtung, die eine Netzhülse gemäß einem der vorhergehenden Ansprüche beinhaltet, und eine Vorrichtung, die zur Einführung in die vaginale, rektale, nasale bzw. bukkale Öffnung angepasst ist.

20. Vorrichtung gemäß Anspruch 19, wobei der Körper des Hilfsmittels aus einem absorbierenden Material hergestellt ist.

21. Vorrichtung gemäß Anspruch 20, wobei das absorbierende Material Cellulose oder Cellulosederivatfasern, Baumwolle, Stärke, Reyon, Schwamm, Holzzellstoff, Polyolefin, Polyester, Polyamid, Polyurethan, vernetzte Carboxymethylcellulose, Acrylsäure, Methacrylsäure, 2-Acrylamido-2-methylpropansulfonsäure oder eine Mischung daraus oder ein Hydrogel ist/sind.

22. Vorrichtung gemäß einem der Ansprüche 19 bis 21, wobei das Hilfsmittel ein Tampon ist.

23. Vorrichtung gemäß einem der Ansprüche 19 bis 22, die ferner ein Einführungsmittel beinhaltet.

24. Vorrichtung gemäß Anspruch 23, wobei das Einführungsmittel ein erstes hohles zylinderförmiges Röhrchen, das einen Einsatz zum Aufnehmen der Vorrichtung definiert, und ein zweites hohles zylinderförmiges Druckstück, das gleitbar innerhalb des ersten zylinderförmigen Röhrchens aufgenommen wird, beinhaltet.

25. Netzhülse gemäß einem der Ansprüche 1 bis 18 oder Vorrichtung gemäß einem der Ansprüche 19-24 zur Verwendung in der Therapie.

## Revendications

1. Un manchon en maille adapté pour être utilisé avec un dispositif convenant pour être inséré dans une cavité corporelle et préparé séparément dudit dispositif de telle sorte que, à l'utilisation, le manchon enveloppe le corps du dispositif, et où le manchon comprend un agent pharmaceutique disposé sur celui-ci.

2. Un manchon selon la revendication 1, où le manchon est adapté pour être utilisé avec un dispositif convenant pour être inséré dans la cavité vaginale, rectale, nasale ou buccale.

3. Un manchon en maille selon la revendication 1 ou la revendication 2, où ledit manchon a une extrémité ouverte et une extrémité substantiellement fermée.

4. Un manchon en maille selon la revendication 1 ou la revendication 2, lequel est ouvert aux deux extrémités.

5. Un manchon en maille selon n'importe laquelle des revendications 1 à 4, où ledit manchon en maille peut se dilater lorsque le dispositif se dilate au lors de l'utilisation.

6. Un manchon en maille selon la revendication 5, où la capacité à se dilater est conférée par la présence d'un chevauchement de matériau en maille.

7. Un manchon en maille selon la revendication 5, où la capacité à se dilater est conférée par l'élasticité du matériau en maille.

8. Un manchon en maille selon la revendication 5, où la capacité à se dilater est conférée par une combinaison de l'élasticité du matériau en maille et la présence d'un chevauchement de matériau en maille.

9. Un manchon en maille selon n'importe laquelle des revendications 1 à 8, lequel comprend un composant d'assujettissement convenant pour attacher le manchon au corps du dispositif.

10. Un manchon en maille selon n'importe laquelle des revendications 1 à 9, où le matériau à partir duquel le manchon en maille est réalisé est du coton, tel que du coton non mouillable.

11. Un manchon en maille selon n'importe laquelle des revendications 1 à 10, auquel sont attachés des coupons pharmaceutiques discrets au nombre de 1, 2, 3, 4, 6, 8, 10, 20, 50, 100 ou plus.

12. Un manchon en maille selon n'importe laquelle des revendications précédentes, où ledit agent pharmaceutique est formulé avec un ou plusieurs excipients et/ou vecteurs acceptables d'un point de vue pharmaceutique.

13. Un manchon en maille selon la revendication 12, comprenant de plus un agent mouillant.

14. Un manchon en maille selon n'importe laquelle des revendications précédentes, où ledit agent pharmaceutique se présente sous la forme d'une composition à libération prolongée.

15. Un manchon en maille selon n'importe laquelle des revendications précédentes, où la quantité d'agent pharmaceutique disposé sur la surface est entre 10 µg et 1 g.

16. Un manchon en maille selon n'importe laquelle des revendications 1 à 15, où cedit ou cesdits agents pharmaceutiques sont sélectionnés parmi un ou plusieurs éléments du groupe consistant en un agent anti-fibrinolytique, tel que de l'acide tranexamique ou de l'acide aminocaproïque ; un agent anti-inflammatoire, tel que de l'ibuprofène ou de l'acide méfénamique ; un agent tocolytique, tel que de la hyoscine ou de la ritodrine ; un agent modificateur d'hémostase ; un stéroïde sexuel ; un glucocorticoïde; un minéralocorticoïde et des compléments alimentaires.

17. Un manchon en maille selon n'importe laquelle des revendications 1 à 16, où ledit dispositif est un dispositif intra-nasal ou un dispositif intra-rectal, et où cedit ou cesdits agents pharmaceutiques sont sélectionnés parmi un ou plusieurs éléments du groupe consistant en adrénaline, nitroprussiate de sodium, antiémétiques, tels que de l'ondansétron, antimigraineux, tels que du sumatriptan, un bronchodilatateur tel que du salbutamol ou de la théophylline, ou diurétiques tels que du frusémide.

18. Un manchon en maille selon n'importe laquelle des revendications 1 à 17, où ledit manchon en maille est attaché à un support.

19. Un appareil intra-vaginal, intra-rectal, intra-nasal ou intra-buccal comprenant un manchon en maille selon n'importe laquelle des revendications précédentes, et un dispositif adapté pour être inséré jusque dans le vagin, le rectum, la cavité nasale ou buccale, respectivement.

20. Un appareil selon la revendication 19, où le corps du dispositif est réalisé à partir d'un matériau absorbant.

21. Un appareil selon la revendication 20, où ledit matériau absorbant est de la cellulose ou des fibres dérivées de cellulose, du coton, de l'amidon, de la rayonne, de l'éponge, de la pâte de bois, de la polyoléfine, du polyester, du polyamide, du polyuréthane, du carboxyméthylcellulose réticulé, de l'acide acrylique, de l'acide méthacrylique, de l'acide 2-acrylamido-2-méthyl propane sulfonique ou un mélange de ceux-ci, ou un hydrogel.

22. Un appareil selon n'importe laquelle des revendications 19 à 21, où ledit dispositif est un tampon.

23. Un appareil selon n'importe laquelle des revendications 19 à 22 comprenant en outre un moyen d'insertion.

24. Un appareil selon la revendication 23, où ledit moyen d'insertion comprend un premier tube cylindrique creux définissant une cartouche destinée à recevoir ledit dispositif et un deuxième piston cylindrique creux reçu par coulissement au sein dudit premier tube cylindrique.

25. Le manchon en maille selon n'importe laquelle des revendications 1 à 18 ou l'appareil selon n'importe laquelle des revendications 19 à 24 destiné à être utilisé en thérapie.
